# EUROPEAN PATENT SPECIFICATION

(11) **EP 2 663 251 B1**
(45) Date of publication and mention of the grant of the patent: **29.10.2014**
(21) Application number: 11709195.9
(22) Date of filing: 11.01.2011
(51) Int. Cl.: A61B 18/24

(54) **LASER SURGERY DEVICE**
LASERCHIRURGIEVORRICHTUNG
DISPOSITIF DE CHIRURGIE AU LASER

(43) Date of publication of application: 20.11.2013
(73) Proprietor: Quanta System S.p.A., 21058 Solbiate Olona VA (IT)
(72) Inventor: FAGNANI, Filippo, I-21058 Solbiate Olona VA (IT)
(74) Representative: Romano, Giuseppe
(86) International application number: PCT/IT2011/000006
(87) International publication number: WO 2012/095873

(56) References cited:
- EP-A1- 0 717 965
- EP-A1- 1 072 231
- WO-A2-93/25136
- WO-A2-2010/014224
- US-A- 5 041 108
- US-A- 5 766 163
- US-A- 6 113 589
- US-A1- 2009 287 197

## Description

### TECHNICAL FIELD

The present invention relates to an accessory for a medical device and also to a medical device comprising said accessory, for the cutting, ablation, resection, vaporization, coagulation and haemostasis of a tissue. In particular, the accessory, and consequently the device which includes it, operates by means of laser radiation.

### STATE OF THE ART

In surgery, the device used for the cutting, ablation and/or haemostasis of tissue is the electrosurgical scalpel, commonly used in operating theatres to overcome the problems arising from the loss of blood caused by the cutting of blood vessels and capillaries. The electrosurgical scalpel makes use of the heat produced by Joule effect by the flow of current at radio frequency. The temperature increase is a function of the power density and the application time and it may reach a level sufficient to overheat the tissue and create a coagulation or cutting effect. If this apparatus is correctly used, it creates only thermal effects in the tissue, while the electrolytic effects are negligible. The thermal effect of the current on the tissue can lead to different transformations in its component cells, depending on the temperature which is reached.

If the temperature is below 100°C, the water contained in the cells is heated, leading to cell concatenation phenomena which cause the blocking of blood loss, while, if it is above 100°C, the result is cell destruction and consequently the cutting of the tissue; finally, if the temperature is much higher than 100°C, the tissue is carbonized, producing a form of coagulation known as cauterization.

In particular, an electrosurgical loop, operated by a device known as a resector, is used in some endourological procedures, such as benign hypertrophic prostate resection or the removal of vesical neoplasia.

The term "resector" is commonly used to signify the set of instruments used for tissue resection; the resector is normally composed of five parts, namely:
- **Optical system:** This may have a variable gauge and angle of view. The optical system is introduced into the instrument through the inner sheath. It is used for viewing and is also connected to a light source and therefore designed to convey visible radiation to illuminate the internal tissues to be examined. The eyepiece is located externally and is commonly connected to a video camera which transmits the images to a suitable monitor. Direct viewing by the surgeon through the eyepiece is now no longer used.
- **Loop:** This is the actual surgical instrument of the resector with which various actions are carried out. The loop may be hot or cold, and may be:
   1. An angled cutting loop, which is most commonly used in the resection of polyps and myomas and in endometrial ablation;
   2. An equatorial cutting loop, which can be used for frontal cutting and for reaching small neoformations in places (such as tube recesses) which could not be reached otherwise;
   3. A scalpel cutting loop for lysis of septums or synaechiae;
   4. A roller ball, roller barrel or toothed roller ball for vaporization, these being spherical or cylindrical, smooth or toothed electrodes used with a current for cutting purposes only in endometrial ablation, minor polypectomy and myomectomy.
- **Electrotome, or operating element, or translation device.** This is the element which is held by the operator. It has active and passive movements for advancing and retracting the loop connected to it, enabling the progress of the loop to be controlled by the use of the thumb.
- **Inner sheath:** This part has the loop inserted into it. It has a valve for the admission of liquid to perform continuous washing of the optical system and allow constant viewing. At the distal end it has a porcelain seal to prevent current loss in case of any accidental contact with the loop.
- **Outer sheath:** This is placed over the inner sheath so as to form a gap which collects liquid through the slits and conveys it to the outside through a discharge valve.

Additional parts are:
1. A liquid delivery tube
2. A liquid outlet tube
3. An electrosurgical scalpel with cable and terminal plate
4. A light cable connected to the light source
5. A telecamera.

Transurethral resection of the prostate, known by the abbreviation TURP, is the most common endoscopic method for removing the obstruction to the emptying of the bladder caused by the prostate. It is indicated in cases of benign prostate pathology and in obstructive malignant neoplasia which is not responsive to hormone treatment and where radical treatment is impossible. It is performed by introducing an instrument known as a resectoscope through the urethra into the deepest part of the prostate, which is then removed, using the electrocoagulation caused by the electrosurgical scalpel for the septation of the tissues. The operation is performed under regional or general anaesthesia and takes 20 to 60 minutes. After the operation, a bladder catheter is fitted, with a system for continuously washing the bladder; this is removed, on average, after three days if there are no complications. The average period of hospitalization is four to five days, according to the official statistics of the Italian Ministry of Health. However, the TURP procedure has the disadvantage of side effects, usually in the form of urinary disorders (often transitory, but recurring) and retrograde ejaculation. The TURP procedure is associated with considerable bleeding during the operation and the recognized complications include haemorrhages (about 7%) requiring transfusions. Because of the high probability of considerable bleeding, bags of autologous or homologous blood are usually prepared; the treatment is particularly complicated, or impracticable, in patients receiving anticoagulant therapy or with serious coagulation problems, or those who cannot accept transfusions because of their religious faith.

Other complications may include infection of the urinary passages (15%) and the vas deferens. On discharge, a period of rest of about 10 days is recommended, with a follow-up examination by urine culture, PSA testing and transrectal prostate echography if necessary.

Various alternative methods have been proposed for use in combination with, or in place of, TURP in order to overcome the problems associated with it. For more than 10 years, certain laser devices, using different wavelengths, operating methods and power levels, have been proposed as alternatives to electrosurgical scalpels in the general surgical field and for the specific TURP procedure in urology.

Resection using an electrosurgical scalpel is still preferred to the proposed alternative methods, owing to the lengthy and difficult training required for these methods, and for financial reasons.

Brief descriptions of the features of the aforementioned lasers, according to their emission wavelengths, are given below for the sake of completeness.

### 980-1064 nm

High-power Nd:YAG lasers emitting at 1064 nm and diode lasers emitting at 980 nm have been used in surgery, for example for vaporization of the prostate. Because of the low blood and water absorption factors at the 1064 nm wavelength, the result is negligible vaporization, combined with significant necrotic phenomena and deep coagulation effects. Postoperative consequences associated with the use of Nd:YAG lasers are urine retention, irritating micturition symptoms and serious infections. The deep coagulation effect and the residual necrotic tissue also lead to the obstruction of the neck of the bladder a few weeks after the operation. For the above reasons, the use of Nd:YAG lasers (at 1064 nm) and diode lasers (at 980 nm) is being discontinued (L.M. Ramos, High Power 980 nm Diode Laser: Preliminary Results in the treatment of benign prostatic Hyperplasia, Arch. Esp. Urol. 2009; 62: 125-130; Malte Rieken et al., Complications of laser prostatectomy: a review of recent data, World J Urol 2010 28:53-62).

### 532 nm

There has been a subsequent move towards the use of frequency doubled Nd:YAG lasers emitting at 532 nm, with high output power. These laser devices, such as that described in US 6986764, are based on the intra-cavity doubling of repeated Q-switched Nd:YAG laser beams, which are then coupled in side firing optical fibres, which are more costly and complicated than front firing fibres. The radiation at the 532 nm wavelength is preferentially absorbed by haemoglobin Hb and HbO₂ and only to a very small extent by water. The radiation absorption causes a local temperature increase which in turn vaporizes the tissue with negative residual coagulation effects which are smaller than those associated with the use of a 1064 nm wavelength (Bach T et.al., RevoLix vaporesection of the prostate: initial results of 54 patients with a 1-year follow-up. World J Urol. 2007; 25:257-62.). Instruments emitting at 532 nm, which originally only provided very slow vaporization, have now had their power increased to 180 W in order to provide operating times similar to those of TURP. Vaporization of a tissue using laser sources which emit in the infrared range and preferentially target interstitial water instead of haemoglobin offers a more comfortable method for patients, which is free of negative effects such as postoperative bruising and/or swelling.

The radiation emitted in the proximity of the peak absorption of water, at about 2 µm, has an absorption length of < 0.5 mm, making it possible to operate with a high volumetric power density if the laser output power is sufficiently high, thus achieving rapid vaporization of the tissue.

This radiation can also be used effectively on tissues without a particularly high level of vascularisation.

### 1470 nm

Lasers emitting at 1470 nm can be used in conditions of lower volumetric power density, because their radiation is absorbed better by water than that of 532 nm lasers, thus improving haemostasis, but decreasing the ablation/vaporization effect.

The haemostatic properties of lasers emitting in the infrared, particularly at 1470 nm, according to Wezel et al. (Felix Wezel et al., New alternatives for laser vaporization of the prostate: experimental evaluation of a 980-, 1,318- and 1,470-nm diode laser device, World J Urol 2010, 28:181-186), and used on the kidneys of pigs, are similar to those found at the 980 nm and 1318 nm diode wavelengths, but are much greater than those found at 532 nm. According to this study, the bleeding fraction is 0.24 ± 0.05 g/min for 1470 nm, whereas it is 0.27 ± 0.08 g/min for 980 nm and 0.35 ± 0.17 g/min for 1318nm, which is considerably better than the amount of bleeding for the 532 nm wavelength (0.65 ± 0.26 g/min). As regards the necrotic areas, these are much less deep for 980 nm and 1318 nm than when 1470 nm radiation is used.

Furthermore, the use of the 1470 nm laser does not cause postoperative haemorrhage or bleeding (Seitz M et al., Ex vivo and in vivo investigations of the novel 1470 nm diode laser for potential treatment of benign prostatic enlargement, Lasers Med Sci 2009, 24:419-424).

As mentioned in US 6986764, coagulation and vaporization require different levels of volumetric power density applied to the tissue. The applied volumetric power density parameter can easily be controlled if the absorption factor is relatively low and the absorption length is sufficiently great.

In present-day practice, attempts are made to achieve the haemostatic effect by reducing the power output of the laser, or by using the optical fibre at a greater distance from the target tissue. However, this method does not resolve the problem of achieving satisfactory haemostasis, since the depth of penetration is unchanged and the efficacy is observable only at the surface level. Furthermore, when the laser is used at a greater distance from the target, it is difficult to control the transmitted power, since most of the radiation is absorbed by the water surrounding the tissue.

### 1900-2200 nm

The laser devices used most widely for soft tissue surgery at the present time are high-power lasers with wavelengths of approximately 2 µm. Because of the high power and short absorption length (less than 0.5 mm), it is possible to operate in conditions of high volumetric power density, thus achieving optimal vaporization. In this case, the change from the ablative to haemostatic mode is made, in practice, by modifying the laser output power and consequently the volumetric power density, although the penetration depth cannot be altered. The interaction between the radiation and the tissue is essentially superficial, and it is therefore impossible to increase the irradiated volume in the direction of the beam. Lasers emitting at approximately 2 µm are therefore highly efficacious for surgery, but less so for haemostasis procedures.

### Procedures

In the treatment of benign prostate hypertrophy, or in tumour ablation, the aforementioned laser devices have used hitherto operating methods other than TURP to achieve the reduction or total removal of the hypertrophic tissue or of the body of the tumour. The techniques used in laser procedures can be summarized, for practical purposes, as selective photo-vaporization or ablation of the prostate (e.g. US 20070225696) or enucleation of the hypertrophic lobes which are detached from the prostate capsule and directed into the bladder where they are subsequently crushed and drawn off. Vaporesection is the term used for vaporization and resection carried out simultaneously (Bach T et al., RevoLix vaporesection of the prostate: initial results of 54 patients with a 1-year follow-up World J Urol. 2007; 25:257-62.).

The methods of vaporization, ablation and enucleation of the prostate using lasers differ from ordinary TURP in their practical aspects, requiring a long learning curve and generally needing longer operating times; however, the use of lasers provides considerable postoperative benefits and the more effective haemostasis enables subsequent transfusion to be avoided.

The present invention proposes leaving the basic TURP procedure unchanged, while making use of the experience acquired by operators in the use of ordinary electrosurgical scalpels and resectors and the recent availability of high-power lasers (up to 200 W).

The object of the present invention is therefore to make it possible to carry out laser TURP (referred to below as LaTURP) using a special device, in a method which is compatible with TURP in terms of procedure and mode of use.

LaTURP is carried out by using the aforementioned instrument, known as a resector, which remains unchanged except as regards the loop, which is replaced by the device proposed by the invention. The device comprises one or more laser sources, conveying means and optical fibres for the transmission of the laser beam from said source(s), and guide elements introduced into the inner sheath of the resector, through which optical fibres which terminate at the tip of the device are passed.

The interaction between the laser and the tissue varies greatly with the emission wavelength; the present invention is intended to permit interfacing with any suitably connected laser device, allowing the user to select the laser characteristics to be used according to the pathology and the tissue treated.

The advantages of using a variety of lasers (the choice of the source laser device depends on the operator, as stated above) in combination with the commonly used TURP procedure are such that training times can be reduced and evident surgical benefits obtained.

The present invention can be used with ordinary resectors available on the market, if suitable interfaces are provided, or with dedicated resectors. The device according to the present invention can be used in surgery, particularly in surgical procedures involving incision and/or vaporization and/or vaporesection and/or ablation and/or enucleation of a target tissue. Preferably, said tissues are soft tissues such as prostate, liver, kidney, or lung tissue, or the like. Even more preferably, said tissue is prostate tissue.

The laser devices described in the prior art and currently used in surgical practice, particularly in soft tissue surgery for the purposes of ablation, vaporization and/or coagulation, are based on emission by a single optical fibre which may be front firing (front fibre) (e.g. US 5416878) or side firing (side fibre) (e.g. US 545668 and US 5486171) or diffused emission, or may be connected to an ordinary resector, but only for the purposes of auxiliary haemostasis (US 5312399).

EP 1 072 231 A1 teaches an accessory according to the preamble of claim 1.

The laser devices described in the prior art and currently used in surgical practice, particularly in soft tissue surgery, are also based on the emission of a single wavelength. Using devices designed in this way, i.e. for operation exclusively at a single wavelength, gives rise to various problems as described above. The application of the present invention means that any single or combined wavelength, from laser sources which may be different from each other, can be used.

The emission described in the present invention is provided by means of two optical fibres which can convey different laser sources, thus replicating the action of an ordinary resector. This makes it possible to supply laser emissions simultaneously or alternately at different wavelengths and/or power levels, from different laser sources or from a single laser device, using a splitting element or an element for doubling the single beam.

The present invention proposes to replicate the action of a resection loop by the simultaneous or alternating combined action of two optical fibres aimed at the same area of tissue, by regulating guide elements of said fibres, in order to carry out the vaporization and/or cutting of the tissue. The particular spatial arrangement of the beams also makes it possible to avoid the formation of deep coagulation, by using laser emission at a tangent to the tissue rather than perpendicularly, as would be the case with laterally emitting fibres, commonly known as "side fibres".

The present description therefore relates to an accessory for a medical device for the laser resection of a tissue by endoscopy as defined in claim 1.

The present description also relates to a medical device for the laser resection of a tissue by endoscopy, comprising the accessory described above, as defined in the independent claim 18.

Preferred features of the present invention are described in the corresponding dependent claims.

In particular, the present invention enables minimally invasive surgery to be performed on the tissue in question, with the greatest possible practicality for the surgeon and the greatest possible safety for the patient.

This is because two different wavelengths, for example a wavelength suitable for vaporization and/or cutting and/or ablation and/or enucleation and a wavelength suitable for coagulation, can be used directly in the target tissue, thus reducing the bleeding from affected blood vessels to a minimum, or in any case enabling haemostasis to be performed at any moment if required.

In other words, the abovementioned use in combination with a haemostatic laser has an immediate effect on the tissue, creating fewer risks for the patient and increasing the success rate of surgical operations.

It is also possible to use the same wavelength and make use of the concentrated spatial effect of the energy supplied.

The present invention is intended to be used with satisfactory results in the same applications in which ordinary monopolar or bipolar electrosurgical scalpels are used, particularly in surgical resection techniques. The use of the laser sources in place of ordinary monopolar or bipolar electrosurgical scalpels is intended to improve the effects of ablation, vaporization and haemostasis and operative and postoperative complications, while extending the range of applications to cases in which the electrosurgical scalpel/resection system is not usable.

Further advantages and the features and methods of use of the present invention are made clear by the following detailed description of possible embodiments of the invention, provided as non-limiting examples, with reference to the appended drawings, in which
- Figure 1A is a view of an accessory for a resector of the conventional type;
- Figure 1B is a view of an accessory according to the present invention;
- Figure 2 is a front view of the accessory showing the final laser elements which can be rotated for superficial or deep interaction with the treated tissue;
- Figure 3 shows an accessory according to the present invention, connected to a medical device;
- Figures 4A and 4B are schematic illustrations of the accessory, in a top and side view respectively, with its connections to optical elements and some parts of a medical device;
- Figure 4C is a rear view of an accessory according to the present invention;
- Figure 4D shows optional protective caps for an accessory according to the present invention;
- Figure 4E shows a detail of an accessory according to the present invention;
- Figure 5A shows a first medical device according to the present invention, comprising the accessory;
- Figure 5B shows a second medical device according to the present invention, comprising the accessory;
- Figure 5D shows the device associated with a schematic resector;
- Figures 6A and 6B are schematic views of two possible connections of the accessory to two separate laser sources;
- Figures 7A and 7B are schematic views of three possible connections of the accessory to a single laser source;
- Figures 8A to 8F are graphs showing possible combinations of laser radiation and their modulation;
- Figure 9 is a schematic exemplary view of the insertion of the device through a resector, used for action on prostate tissue;
- Figures 10A to 10D show the use of the device for carrying out, for example, vaporization of the tissue, using a particular inclination and operating distance from the tissue while maintaining an adequate haemostasis and coagulation action;
- Figures 11A to 11D show the use of the device for carrying out, for example, the resection of a tissue, where, using a particular inclination and bringing the tips into contact with the tissue, the translational movement is the same as that used in ordinary TURP; by means of TURP adequate haemostasis and coagulation can also be maintained.

### DETAILED DESCRIPTION OF THE INVENTION

Reference will be made to the above figures in the following description of the present invention.

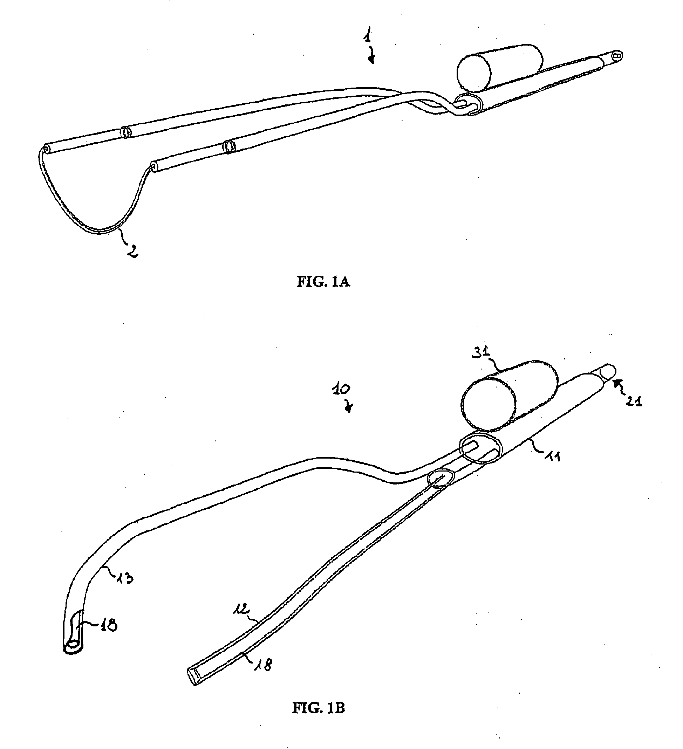

With initial reference to Figures 1A and 1B, the structural similarity between a conventional resector 1 and an accessory 10 according to the present invention is shown, both of these being capable of directly reaching a target tissue with one or more operating tips. The conventional resector 1 acts, as is known to those skilled in the art, in such a way that the operation is performed solely by means of the contact of the loop 2 with the tissue concerned.

One of the advantages of the present invention, which will be made clear by the following description, is that it can act even when it is not in contact with the target tissue. In particular, a first subject of the present description is an accessory 10 designed to simulate the action of the resector loop, while allowing the operator to act also at a distance not close to the tissue.

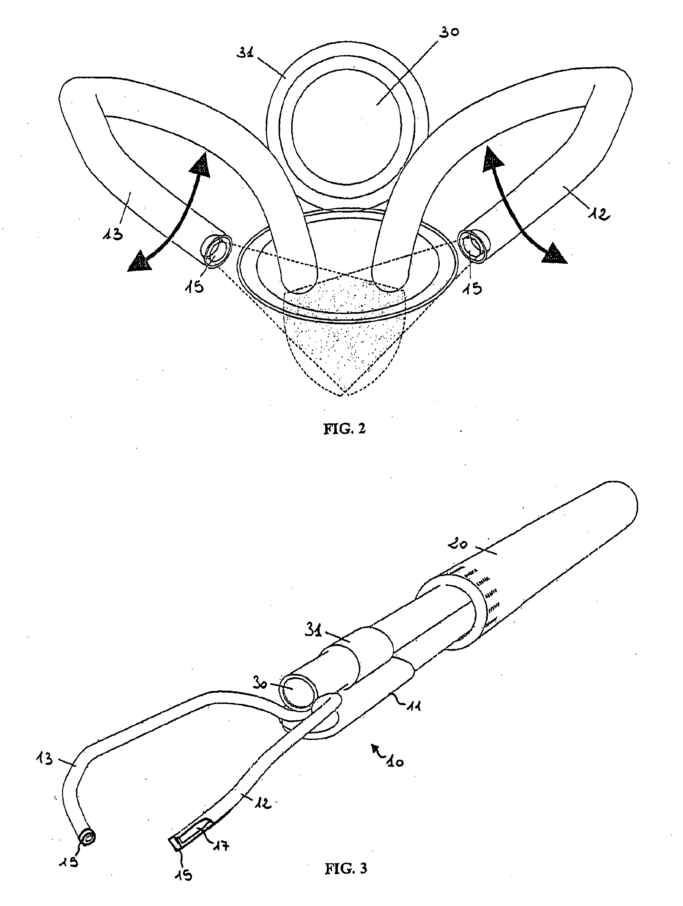

The subsequent Figures 2 to 4B are views of an accessory 10 according to the present invention.

The accessory 10 can be used to optimize the processes for cutting, ablation, resection, vaporization of the tissues and simultaneously for the effective control of the haemostasis of the blood vessels which may be damaged during the operation.

The accessory 10 according to the present invention is therefore an accessory for a medical device in which said device is suitable for the laser cutting, ablation, resection, vaporization and/or coagulation of a tissue, preferably by endoscopy.

The accessory 10 comprises, in the first place, a supporting element 11 and at least two guide elements 12 and 13.

The guide elements 12 and 13 are capable of carrying corresponding optical fibres, preferably one optical fibre in each guide element.

As shown in the drawings, the guide elements 12 and 13 are mounted on the supporting element 11 which can be connected to a medical device 20. This connection is provided by the presence of means 21 for connecting the support 11 to the medical device 20.

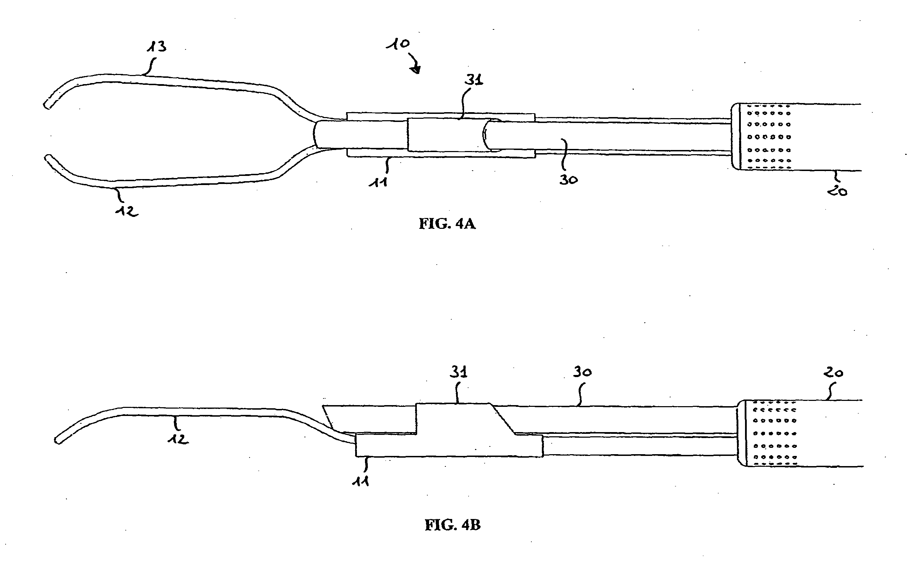

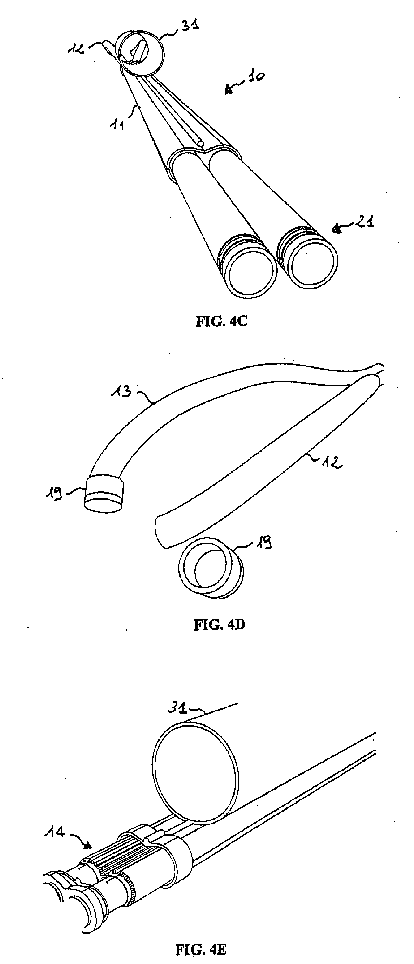

These connecting means 21 may include - but are in no way limited to-engaging, sliding, bayonet-type or male and female systems. Figure 4C, which is a rear view of an accessory according to the present invention, shows by way of example a bayonet-type connection. Clearly, connecting means which are not explicitly described herein, but which are considered suitable by persons skilled in the art for ensuring cooperation between the accessory 10 and a medical device using the support 11, are to be considered as part of the present description.

As stated above, the accessory 10 is preferably used in the surgical procedures of laser cutting, ablation, resection, vaporization and/or coagulation of a tissue, preferably performed by endoscopy. For this reason the accessory 10 may further comprise first optical means 30 for endoscopic viewing of the target tissue. These first optical means 30 can be placed inside a first housing 31 in order to align the endoscopic view with the resection area and protect the optical elements from direct laser radiation which would damage them.

A person skilled in the art will be able to determine which optical means should be used in the construction of the accessory 10 described herein, as well as determine the type of housing most suitable for them. By way of non-limiting example, these optical means 30 may comprise at least one lens and/or an objective.

The accessory 10 is apt to carry at least two optical fibres 17 and 18. These fibres, which are preferably sterile, can be mounted inside the guide elements 12 and 13. Clearly, the presence of at least one entrance to the guide elements allows the optical fibres to be introduced into the guide elements. In other words, in the preferred embodiment of the accessory 10, these guide elements 12 and 13 are hollow tubular elements.

The function of the optical fibres is to convey laser radiation to the target tissue or site. The length and thickness of the tubular elements can therefore be determined easily by a person skilled in the art, without the need for further details, according to the structural characteristics of the fibres chosen as being suitable for the embodiment of the present invention.

The accessory 10 may also comprise locking elements 16 for each of the optical fibres 17 and 18. These securing elements 16 allow the optical fibres to be locked after their insertion into the guide elements, thus preventing them from accidentally sliding. This is because excessive sliding would expose the fibres to rapid deterioration, due, for example, to continual direct contact with the tissue and/or with the guides. The integrity of the fibres is important in order to maintain optimal functionality of the resection accessory 10.

As shown in Figure 4D, the accessory 10 according to the present invention may advantageously comprise protective elements 19, similar to caps for example, which can be fitted on the operating tip of each guide element 12 and 13. These caps 19 may be advantageously used during the operations of transport and/or autoclaving, and, more generally, whenever the instrument is to be protected. They also allow precise definition of the degree to which the fibres are inserted into the elements. Each optical fibre can therefore be inserted, when its protective element has been applied to the guide element, until it touches said element, and can then be fixed by means of suitable securing devices which will be described more fully below. The dimensions, characteristics and materials of the protective elements 19 can be determined by a person skilled in the art according to the purposes of the operation.

The accessory 10 is designed essentially for use in the medical field and it is therefore preferable that it should satisfy certain requirements, such as that of sterility. For this purpose, at the end of each treatment the optical fibres may be removed for sterilization or for replacement with new fibres to be used for the next treatment.

Additionally, in order to improve the effect on the tissue and thus achieve effective processes of cutting, ablation, resection, vaporization and/or coagulation, it is advantageously possible to provide means for regulating the position, and particularly the inclination, of one or more optical fibres. In one embodiment of the present invention, the accessory 10 may comprise first means for regulating the position of at least one guide element with respect to the tissue.

In the construction of the device, a person skilled in the art may use any regulating means or system which enable at least one guide element to be positioned differently according to the requirements of use. By way of a non-limiting example these first regulating means (not shown in the drawings) may include pairs of rotating collars which, acting on one or both of the guide elements, may modify their positions. Advantageously, these first regulating means can be integrated into the securing elements 16, 16' and/or integrated into the elements 12, 13 themselves. Figure 4E shows, by way of example, a possible embodiment of these first regulating means, integrated with the guide elements 12, 13 and provided in the form of rotating collars 14.

Since the focusing of the laser emission can be regulated by regulating the position and/or inclination of the guide elements, it is possible to regulate the effective distance from the tissue, thus enabling the instrument to be used both in contact with the tissue and at a distance. Suitable arrangements are provided on the output elements to protect the tips of the delivery fibres. However, it is considered unnecessary to describe these arrangements in greater detail, as they will be evident to persons skilled in the art.

In one embodiment of the present invention, a lens 15 may also be provided at an operating end of each of the guide elements 12, 13. This lens 15, preferably of the converging type, is useful for concentrating the laser radiation in the proximity of the target tissue.

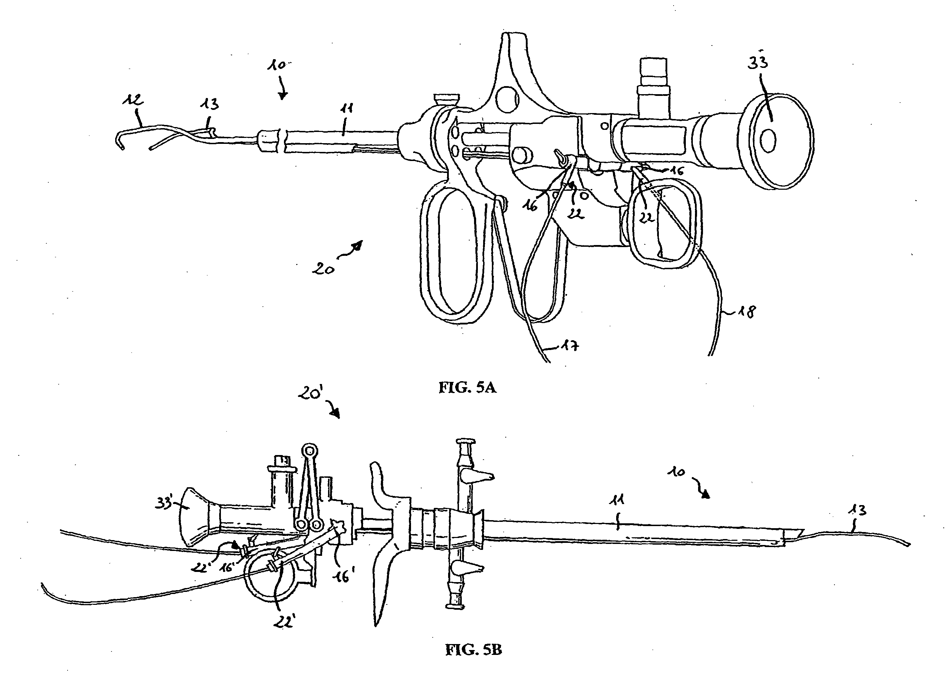

The subsequent Figures 5A and 5B relate to two medical devices 20, 20' according to the present invention, each provided with an accessory 10 as described hitherto.

In particular, two medical devices 20, 20' for the laser cutting, ablation, resection, vaporization and/or coagulation of a tissue, preferably by endoscopy, are shown. The devices 20 and 20' are characterized by the presence of an accessory 10 as described above and at least one interface element which can co-operate with the connecting means 21 present in the accessory. A person skilled in the art will be able to design the interface element of the device in accordance with the type of connecting means which are included in the accessory 10 and with which the element is required to co-operate.

Each of the devices 20 and 20' comprises two inputs 22, 22' for the optical fibres, located on the main body of the device. Securing elements 16, 16' as described above may be provided at the inputs 22, 22'.

Advantageously, the medical devices 20 and 20' may also be provided with second regulating means for controlling and/or defining the inclination of at least one of the guide elements 12, 13 included in the accessory 10. These second regulating means may be considered to be structurally and functionally similar to the first regulating means, as described above, included in the accessory 10.

The devices 20 and 20' are essentially designed for use in surgical procedures, preferably by endoscopy. The viewing of the target site and/or tissue can be facilitated by the presence of second optical means suitably connected to the eyepiece 33, 33' of the first means 30. These optical means may comprise, for example, lenses and/or objectives and/or video cameras.

The structure and further technical details for the construction of devices according to the present description, of which the devices 20 and 20' are only some of the possible embodiments, are well known to persons skilled in the art and therefore do not need to be detailed more fully in this description.

The laser radiations to be conveyed by the optical fibres 12 and 13 are emitted by one or more laser sources, as will be readily understood. These laser sources may be advantageously incorporated in the accessory 10.

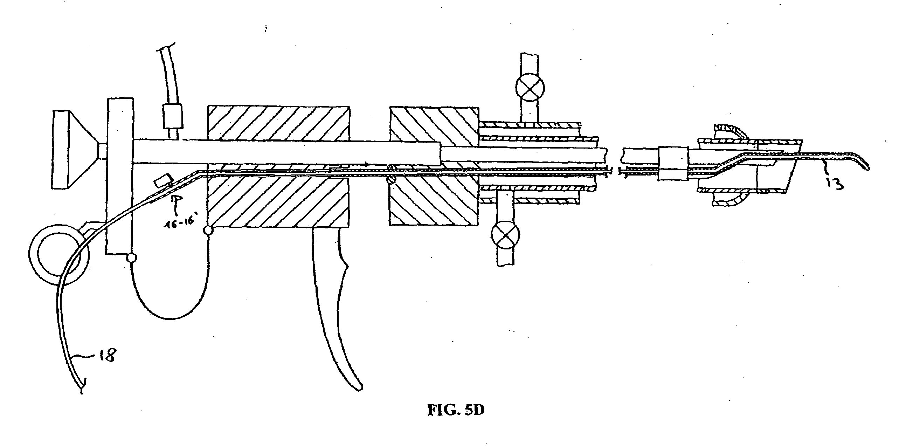

Figure 5D shows the accessory associated with a schematic resector. The optical supply devices 17, 18 depart from one or more laser sources and terminate at the tip of the device which focuses their emission beam. Said fibres are fixed by the elements 16, 16' and are carried and protected by the elements 12, 13. The whole instrument can be introduced and retracted as required by the operator, using the translation device. This operation can also be carried out by moving the whole instrument without using the resector. It is also possible to produce instruments completely dedicated to LaTURP.

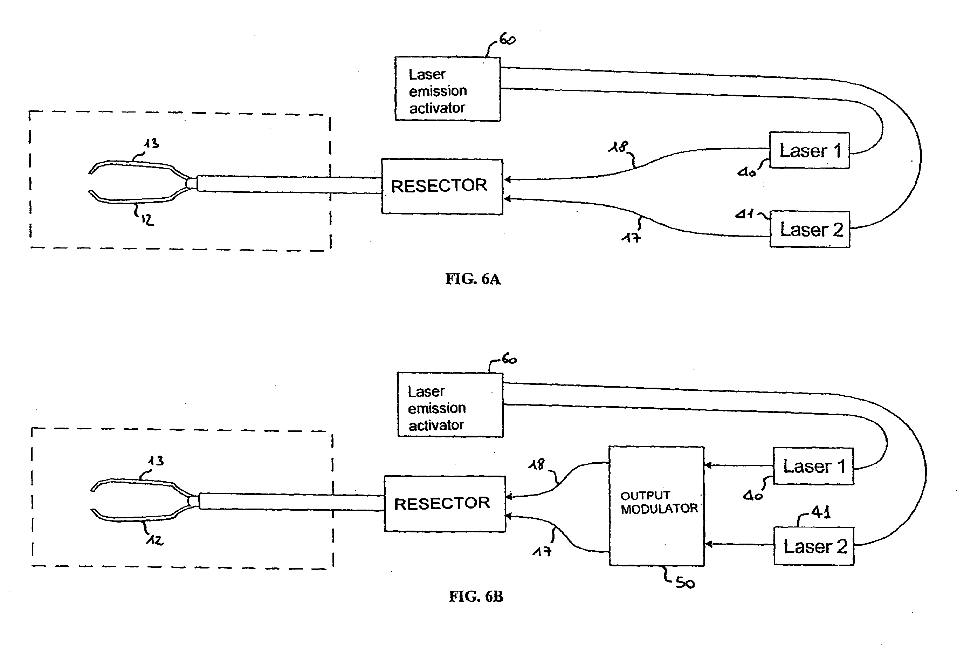

With reference now to Figures 6A and 6B, these show, in schematic form, two possible connections of the accessory according to the present invention to two different laser sources 40, 41.

The device is intended to be used with laser sources emitting beams at any wavelength, with any energy, in continuous or pulsed emission.

With reference now to Figures 6A and 6B, these show, respectively, a first constructional layout of the present invention, in which two separate laser sources 40, 41 are used.

In particular, according a first embodiment, shown in Figure 6A, the accessory 10 may comprise a first source 40 for emitting a first laser radiation and a second source 41, separate from the first, for emitting a second laser radiation. Each of said first and second sources supplies a respective one of the two optical fibres 17 and 18.

In order to activate selectively one or other or both of the laser sources 40, 41, an activator of the laser emission 60 may advantageously be provided.

The specific embodiment of this activator can be chosen freely by a person skilled in the art. For example, it may comprise one or more pedals by means of which the surgeon can select the laser source to be activated. For example, the activator may permit the following combinations:
- (first optical fibre 17)/(first laser source 40) and (second optical fibre 18)/(second laser source 41);
- (first optical fibre 17)/(first laser source 40) and (second optical fibre 18)/(first laser source 40); or
- (first optical fibre 17)/ (second laser source 41) and (second optical fibre 18)/ (second laser source 41).

Thus the operator may, at his/her discretion, combine sources which emit radiation with different or uniform effects on the tissue. In fact, the effects of vaporization and/or vaporesection and/or cutting and/or ablation and haemostasis (coagulation) may be necessary during surgical operations, as already pointed out above. Accordingly, the presence of two sources makes it possible to carry out cutting, ablation, resection and vaporization by using one specific radiation and coagulation of the target tissue by using the other radiation.

For example, purely by way of example, the first laser radiation may have a wavelength close to the water absorption peak, particularly within the spectral region from 1900 to 2100 nm and with a variable power of up to 200 W. The first laser radiation may operate in either continuous or pulsed mode.

This choice is preferable, in particular, when vaporesection of the target tissue must be performed.

The radiation-tissue interaction in the 1900-2100 nm spectral region essentially takes place by the absorption of the water. The penetration length is extremely small, at L1 <0.5 mm.

When the volumetric power density levels are sufficiently high, simultaneous vaporization and resection (vaporesection) of the tissue takes place.

When the vaporization rate is high, bubbles are present, indicating the high effectiveness of the treatment. The vaporization rate can also be controlled by varying the angle of incidence of the radiation on the tissue or by suitably modulating the power of the laser source.

Because of the high power and short absorption length (less than 0.5 mm), it is possible to operate in conditions of high volumetric power density, thus achieving effective vaporization.

The second laser radiation may, for example, have a wavelength close to the water absorption peak in the spectral region from 1400 nm to 1540 nm, with an output volumetric power density which promotes the haemostasis process. In this case also, the interaction is with water, but the absorption length is greater (1.5 mm).

Figure 6B shows a second variation of embodiment of the present invention, again using two separate laser sources 40, 41.

In this case, while the above description is still applicable, the device further comprises a modulator 50 interposed between the outputs of the laser sources 40, 41 and the input of the instrument.

This is because, as mentioned above, different stages of operation, such as a cutting stage which may or may not be followed by a stage of haemostasis, can be identified in a surgical operation. However, it might sometimes be necessary, during an operation, to carry out the cutting stage and the haemostasis stage simultaneously. Consequently, the laser radiation conveyed by the optical fibres may, in the accessory according to the present invention, be laser radiations which can be modulated temporally and spatially by any type of modulator in the accessory.

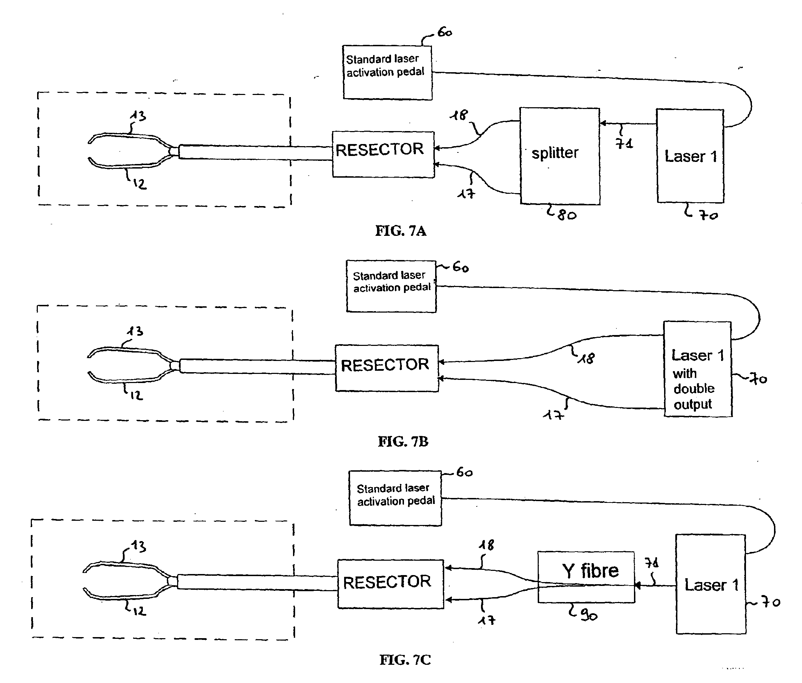

Figures 7A, 7B and 7C show a second constructional layout of the present invention, in which a single laser source 70 is used.

In this case, the device comprises a single source 70 for emitting a laser radiation. The radiation emitted by the single source 70 is then divided between the two optical fibres 17, 18.

The three variants shown in Figures 7A, 7B and 7C show three different methods of dividing the single laser radiation between the optical fibres 17, 18.

In particular, Figure 7A shows the use of a splitter 80, in other words an optical fibre-to-fibre coupler which can divide the radiation from the output optical fibre 71 of the single laser source.

Figure 7B shows the use of a single laser source 70 which, however, already has two separate outputs which can therefore directly supply the two optical fibres 17 and 18.

Figure 7C shows the use of a doubler 90 of the output optical fibre 71 of the laser source, this device being made, for example, in the form of a Y-shaped optical fibre.

The single laser source 70 can be activated by means of the normal actuator provided by the manufacturer.

The laser device according to the present invention is suitable for use in surgery, particularly in surgical procedures for laser cutting, ablation, resection, vaporization and/or coagulation of a tissue. Vaporesection is the term used to refer to vaporization and resection carried out simultaneously (Bach T et al., RevoLix vaporesection of the prostate: initial results of 54 patients with a 1-year follow-up World J Urol. 2007; 25:257-62.). Preferably, said tissues are soft tissues such as prostate, liver, kidney, or lung tissue, or the like. Even more preferably, said tissue is prostate tissue.

The possibility of a continuous-wave (CW) emission mode associated with the first laser radiation enables a precise incision to be made, promoting a significant effect in combination with the vaporization of prostate tissue in urology (T. Back et al., Thullium:YAG 2 micron CW laser prostatectomy, where we stand. World J. Urol.28:163-168; Wendt-Nordhal G., et al., Systematic evaluation of a recently introduced 2-µm continuous-wave Thulium laser for vaporesection of prostate, Journal of Endourology, 22, N. 5, May 2008). In urology, the main treatments in which the device according to the invention can be used are, by way of non-limiting example, benign prostate hyperplasia (BPH), recurrent bladder tumours, contracture of the neck of the bladder, stricture of the urethra and urethral stenosis, and urethral tumours. The diameter of the optical fibres is preferably within a range of 100 to 1000 microns.

Laser radiation may generally be modulated and may therefore be emitted in continuous or alternating mode.

The presence of an emission modulator enables the radiation from the single laser to be divided between both optical fibres carried by the instrument 10 when only one of the emitting sources is suitably activated by the pedal system. When both of the connected laser sources are used simultaneously, each of the fibres carried by the device is intended for the conveying of only one of the two emitting laser sources.

As described above, it is possible to use an emission modulator 50 which, acting on the incoming laser sources, controls the emission modes of each of the single fibres carried in the instrument.

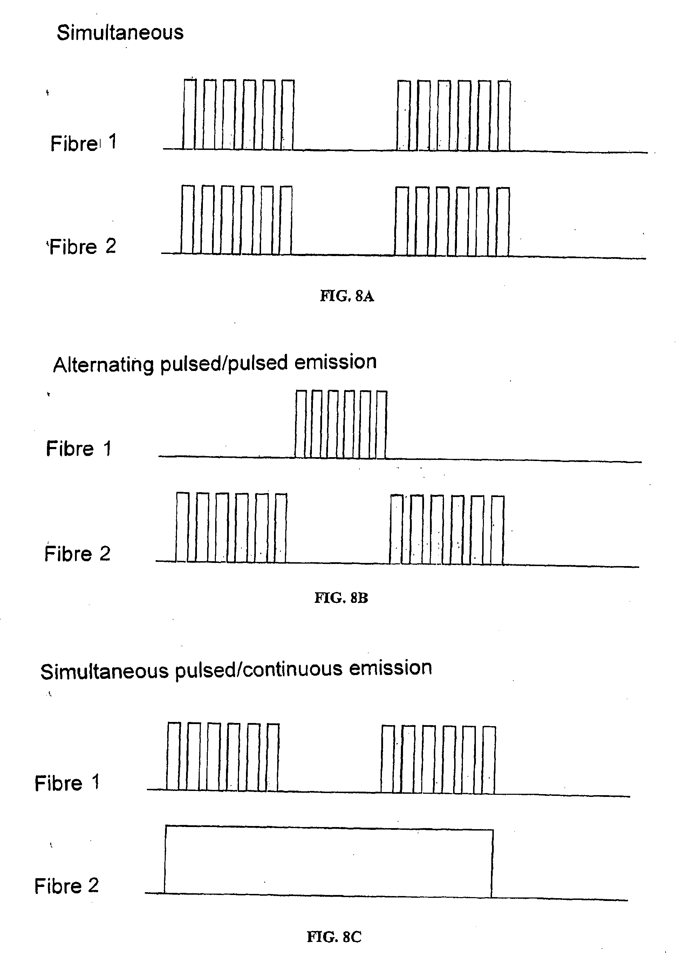

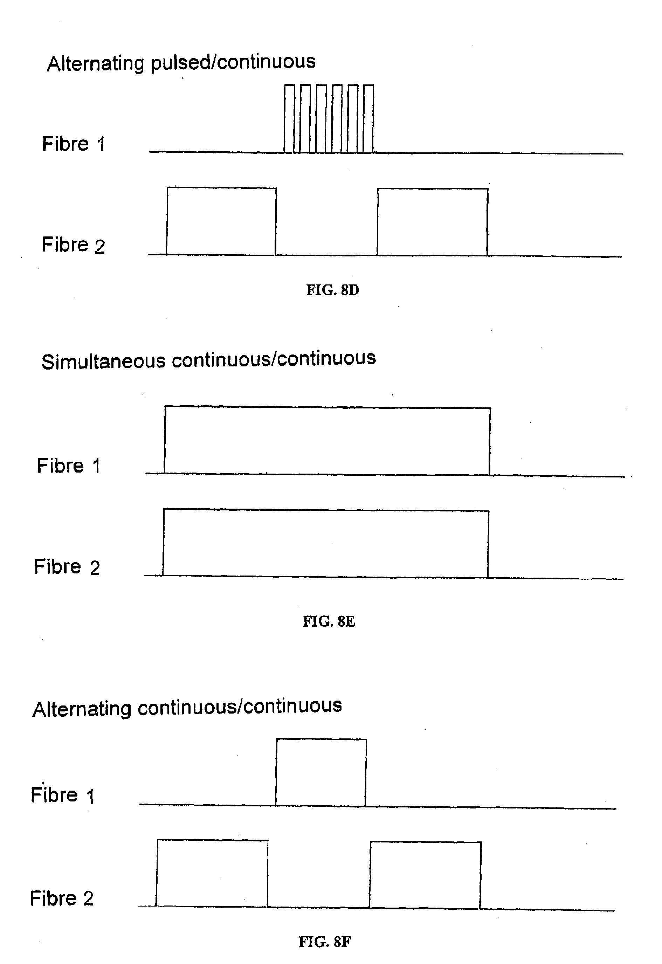

Examples of output combinations are shown in Figures 8A to 8F, for example:
- Simultaneous emission of the terminals of the instrument connected to laser supply sources with pulsed and/or continuous emission.
- Alternating emission of the terminals of the instrument connected to laser supply sources with pulsed and/or continuous emission.
- Continuous emission of the first terminal and alternating emission of the second terminal of the instrument connected to laser supply sources with pulsed and/or continuous emission.
- Alternating emission of the first terminal and continuous emission of the second terminal of the instrument connected to laser supply sources with pulsed and/or continuous emission.

These combinations can produce different types of action on the tissue according to the wavelengths and the power supplied by the individual supply sources. The operating modes of the aforementioned modulator are not described herein because they will be familiar to the average person skilled in the art.

It is to be understood that the modulator may be used in all cases, including the configuration of the invention in which there is a single laser source.

It is also to be understood that, generally, in all the embodiments described above the surgical and/or haemostatic procedures can be carried out with the output optical fibres in contact with or in the proximity of the tissue.

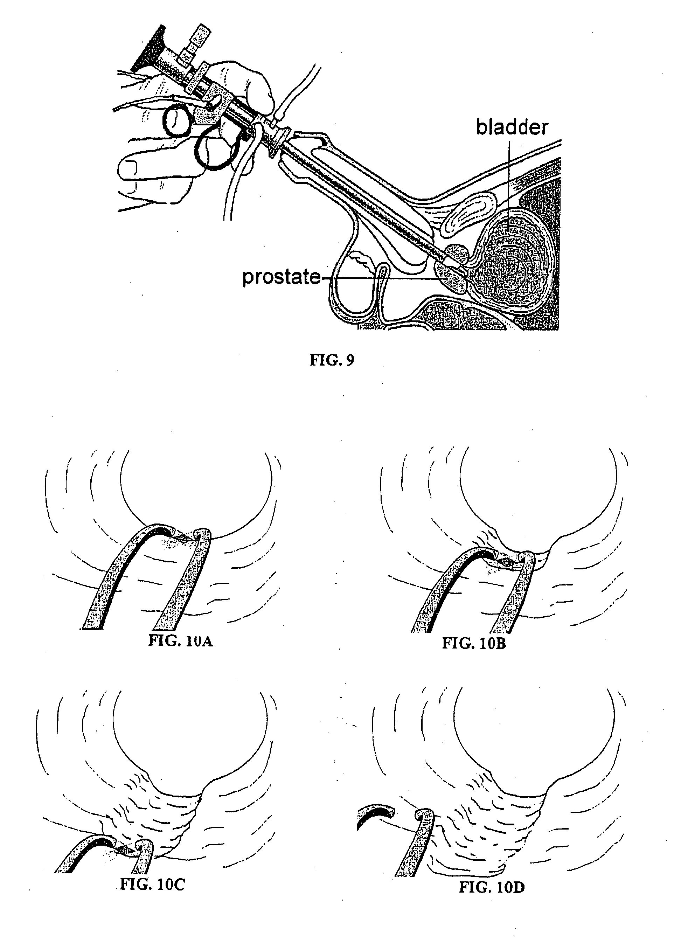

Figure 9 is a schematic exemplary view of the insertion of the device through a resector, used for action on prostate tissue.

The following Figures 10A to 10C show, in sequence, the use of the device for performing, for example, vaporization of tissue, using a particular inclination and distance from the tissue during operation, while maintaining a sufficient degree of haemostasis and coagulation.

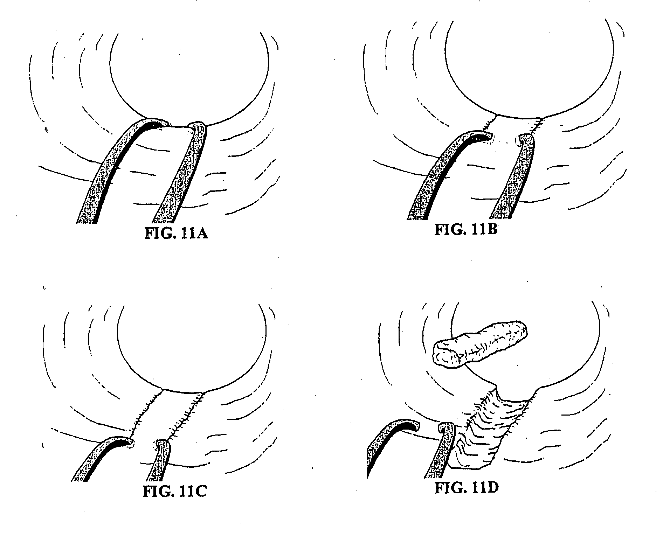

Finally, Figures 11A to 11D show the use of the device for performing, for example, tissue resection, using a particular inclination and bringing the ends of the optical fibres into contact with the tissue, the translational movement being the same as that used in ordinary TURP. By using LaTURP it is also possible to maintain a sufficient haemostatic and coagulating action.

The present invention has been described hitherto with reference to its preferred embodiments. It is to be understood that there may be other embodiments based on the same inventive concept, all such embodiments being included within the scope of the claims provided below.

## Claims

1. An accessory (10) for a medical device (20, 20') for the laser cutting, ablation, resection, vaporization, coagulation and/or haemostasis of a biological tissue, by endoscopy, comprising:
- a supporting element (11);
- two guide elements (12, 13) for carrying respective optical fibres (17, 18), mounted on said support (11);
- first optical means (30) for the endoscopic viewing of said tissue;
- means (21) for connecting said support (11) to said medical device (20, 20'), and
- two optical fibres (17, 18) which are front fibres, wherein each of said guide elements (12, 13) has a first bended projection on a first plane.
**characterised in that** each of said guide elements (12, 13) has a second bended projection on a second plane substantially orthogonal to said first plane.

2. The accessory (10) according to claim 1, wherein said connecting means (21) comprise engaging, sliding, bayonet-type or male and female systems.

3. The accessory according to claim 1 or 2, further comprising a first housing (31) for said first optical means.

4. The accessory (10) according to any one of claims 1 to 3, wherein said first optical means (30) comprise at least one lens and/or an objective lens.

5. The accessory (10) according to any one of claims 1 to 4, wherein each of said guide elements (12, 13) comprises a lens (15) positioned at its operating tip.

6. The accessory (10) according to claim 5, wherein said lens (15) Is of the converging type.

7. The accessory (10) according to any one of claims 1 to 6, wherein said at least two guide elements (12, 13) comprise at least one input (22, 22') for said at least two optical fibres (17, 18).

8. The accessory (10) according to claim 7, further comprising securing elements (16, 16') for said at least two optical fibres (17, 18).

9. The accessory (10) according to any one of claims 1 to 8, further comprising first regulating means (19) for regulating the position of at least one of said guide elements (12, 13) with respect to said tissue.

10. The accessory (10) according to claim 9, wherein said first regulating means comprise pairs of rotating collars (14).

11. The accessory (10) according to any one of claims 1 to 10, further comprising a doubling element (80, 90) having an input for each optical fibre and two outputs for optical fibres.

12. The accessory (10) according to any one of claims 1 to 11, further comprising at least one modulator element (50) for receiving a laser radiation at its input and producing a continuous and/or alternating modulated laser radiation at its output.

13. The accessory according to any one of claims 1 to 12, further comprising at least one laser source (40,41, 70).

14. The accessory according to claim 13, further comprising means (60) for activating said at least one laser source (40, 41, 70).

15. The accessory according to claim 14, wherein said activating means (60) comprise at least one pedal.

16. The accessory according to any one of claims 1 to 15, further comprising a protective element (19) for each of said guide elements (12, 13).

17. The accessory according to claim 16, wherein each of said protective elements (19) is in the form of a cap which is fittable on an operating tip of a respective guide element (12, 13).

18. A medical device (20, 20') for the laser cutting, ablation, resection, vaporization and/or coagulation of a tissue by endoscopy, comprising:
- an accessory (10) according to any one of claims 1 to 17, and
- at least one element for interfacing with said accessory (10).

19. The medical device (20, 20') according to claim 18, further comprising second regulating means for regulating the inclination of at least one of said guide elements.

20. The medical device according to claim 18 or 19, further comprising a translation device.

21. The medical device according to any one of claims 18 to 20, further comprising second optical means for endoscopic viewing.

22. The medical device according to claim 21, wherein said second optical means comprise an eyeplece (33, 33') and/or a lens and/or an objective lens and/or a video camera.

## Patentansprüche

1. Zubehör (10) für eine medizinische Vorrichtung (20, 20') für das Laserschneiden, die Laserabtragung, die Laserresektion, das Laserverdampfen, die Laserkoagulation und/oder die Laserhämostasis eines biologischen Gewebes durch Endoskopie, umfassend:
- ein Stützelement (11);
- zwei Führungselemente (12, 13) zum Tragen jeweiliger optischer Fasern (17, 18), die auf dem Stützelement (11) angebracht sind;
- erste optische Mittel (30) zum endoskopischen Betrachten des Gewebes;
- Mittel (21) zum Verbinden des Stützelementes (11) mit der medizinischen Vorrichtung (20, 20') und
- zwei optische Fasern (17, 18), welche Frontfasern sind, wobei jedes der Führungselemente (12, 13) eine erste gebogene Projektion auf einer ersten Ebene hat,
**dadurch gekennzeichnet, dass** jedes der Führungselemente (12, 13) eine zweite gebogene Projektion auf einer zweiten Ebene hat, die im Wesentlichen senkrecht zu der ersten Ebene ist.

2. Zubehör (10) nach Anspruch 1, wobei die Verbindungsmittel (21) Kontaktsysteme, Gleitsysteme, bajonettartige Systeme oder Männlich/Weiblich-Systeme umfassen.

3. Zubehör nach Anspruch 1 oder 2, das weiterhin ein erstes Gehäuse (31) für die ersten optischen Mittel aufweist.

4. Zubehör (10) nach einem der Ansprüche 1 bi 3, wobei die ersten optischen Mittel (30) wenigstens eine Linse und/oder eine Objektivlinse umfassen.

5. Zubehör (10) nach einem der Ansprüche 1 bis 4, wobei jedes der Führungselemente (12, 13) eine Linse (15) umfasst, die an seiner Arbeitsspitze angeordnet ist.

6. Zubehör (10) nach Anspruch 5, wobei die Linse (15) konvergent ist.

7. Zubehör (10) nach einem der Ansprüche 1 bis 6, wobei die wenigstens zwei Führungselemente (12, 13) wenigstens einen Eingang (22, 22') für die wenigstens zwei optischen Fasern (17, 18) umfassen.

8. Zubehör (10) nach Anspruch 7, das weiterhin Sicherungselemente (16, 16') für die wenigstens zwei optischen Fasern (17, 18) umfasst.

9. Zubehör (10) nach einem der Ansprüche 1 bis 8, das weiterhin erste Reguliermittel (19) zum Regulieren der Position von wenigstens einem der Führungselemente (12, 13) bezüglich des Gewebes umfasst.

10. Zubehör (10) nach Anspruch 9, wobei das erste Reguliermittel ein Paar rotierender Kränze (14) umfasst.

11. Zubehör (10) nach einem der Ansprüche 1 bis 10, das weiterhin ein Verdopplungselement (80, 90) mit einem Eingang für jede optische Faser und zwei Ausgänge für optische Fasern umfasst.

12. Zubehör (10) nach einem der Ansprüche 1 bis 11, das weiterhin wenigstens ein Modulationselement (50) zum Empfangen einer Laserstrahlung an seinem Eingang und zum Erzeugen einer kontinuierlichen und/oder alternierenden modulierten Laserstrahlung an seinem Ausgang umfasst.

13. Zubehör (10) nach einem der Ansprüche 1 bis 12, das weiterhin wenigstens eine Laserquelle (40, 41, 70) umfasst.

14. Zubehör nach Anspruch 13, das weiterhin Mittel (60) zum Aktivieren der wenigstens einen Laserquelle (40, 41, 70) umfasst.

15. Zubehör nach Anspruch 14, wobei die Aktivierungsmittel (60) wenigstens ein Pedal umfassen.

16. Zubehör nach einem der Ansprüche 1 bis 15, das weiterhin ein Schutzelement (19) für jedes der Führungselemente (12, 13) umfasst.

17. Zubehör nach Anspruch 16, wobei jedes der Schutzelemente (19) die Form einer Kappe hat, die auf einer Arbeitsspitze eines jeweiligen Führungselementes (12, 13) aufgesetzt werden kann.

18. Medizinische Vorrichtung (20, 20') für das Laserschneiden, die Laserabtragung, die Laserresektion, die Laserverdampfung und/oder die Laserkoagulation eines Gewebes durch Endoskopie, umfassend:
ein Zubehör (10) gemäß einem der Ansprüche 1 bis 17 und
wenigstens ein Element als Schnittstelle zu dem Zubehör (10).

19. Medizinische Vorrichtung (20, 20') nach Anspruch 18, die weiterhin zweite Reguliermittel zum Regulieren der Neigung von wenigstens einem der Führungselemente umfasst.

20. Medizinische Vorrichtung nach Anspruch 18 oder 19, die weiterhin eine Translationsvorrichtung umfasst.

21. Medizinische Vorrichtung nach einem der Ansprüche 18 bis 20, die weiterhin zweite optische Mittel zur endoskopischen Betrachtung umfasst.

22. Medizinische Vorrichtung nach Anspruch 21, wobei die zweiten optischen Mittel ein Okular (33, 33') und/oder eine Linse und/oder eine Objektivlinse und/oder eine Videokamera umfassen.

## Revendications

1. Accessoire (10) pour un dispositif médical (20, 20') pour la découpe au laser, l'ablation, la résection, la vaporisation, la coagulation et/ou l'hémostase d'un tissu biologique, par endoscopie, comprenant :
un élément de support (11);
deux éléments de guidage (12, 13) pour porter des fibres optiques (17, 18) respectives, montés sur ledit support (11);
des premiers moyens optiques (30) pour la visualisation endoscopique dudit tissu ;
des moyens (21) pour raccorder ledit support (11) audit dispositif médical (20, 20'), et
deux fibres optiques (17, 18) qui sont des fibres avant, dans lequel chacun desdits éléments de guidage (12, 13) a une première projection pliée sur un premier plan,
**caractérisé en ce que** chacun desdits éléments de guidage (12, 13) a une seconde projection pliée sur un second plan sensiblement orthogonal audit premier plan.

2. Accessoire (10) selon la revendication 1, dans lequel lesdits moyens de raccordement (21) comprennent des systèmes de mise en prise, coulissants, de type à baïonnette ou mâle et femelle.

3. Accessoire selon la revendication 1 ou 2, comprenant en outre un premier boîtier (31) pour lesdits premiers moyens optiques.

4. Accessoire (10) selon l'une quelconque des revendications 1 à 3, dans lequel lesdits premiers moyens optiques (30) comprennent au moins une lentille et/ou une lentille d'objectif.

5. Accessoire (10) selon l'une quelconque des revendications 1 à 4, dans lequel chacun desdits éléments de guidage (12, 13) comprend une lentille (15) positionnée au niveau de sa pointe opérationnelle.

6. Accessoire (10) selon la revendication 5, dans lequel ladite lentille (15) est du type convergent.

7. Accessoire (10) selon l'une quelconque des revendications 1 à 6, dans lequel lesdits au moins deux éléments de guidage (12, 13) comprennent au moins une entrée (22, 22') pour lesdites au moins deux fibres optiques (17, 18).

8. Accessoire (10) selon la revendication 7, comprenant en outre des éléments de fixation (16, 16') pour lesdites au moins deux fibres optiques (17, 18).

9. Accessoire (10) selon l'une quelconque des revendications 1 à 8, comprenant en outre des premiers moyens de régulation (19) pour réguler la position d'au moins l'un desdits éléments de guidage (12, 13) par rapport audit tissu.

10. Accessoire (10) selon la revendication 9, dans lequel lesdits premiers moyens de régulation comprennent des paires de colliers rotatifs (14).

11. Accessoire (10) selon l'une quelconque des revendications 1 à 10, comprenant en outre un élément double (80, 90) ayant une entrée pour chaque fibre optique et deux sorties pour les fibres optiques.

12. Accessoire (10) selon l'une quelconque des revendications 1 à 11, comprenant en outre au moins un élément de modulateur (50) pour recevoir un rayonnement de laser au niveau de son entrée et produire un rayonnement de laser modulé continu et/ou alterné au niveau de sa sortie.

13. Accessoire selon l'une quelconque des revendications 1 à 12, comprenant en outre au moins une source de laser (40, 41, 70).

14. Accessoire selon la revendication 13, comprenant en outre des moyens (60) pour activer ladite au moins une source de laser (40, 41, 70).

15. Accessoire selon la revendication 14, dans lequel lesdits moyens d'activation (60) comprennent au moins une pédale.

16. Accessoire selon l'une quelconque des revendications 1 à 15, comprenant en outre un élément de protection (19) pour chacun desdits éléments de guidage (12, 13).

17. Accessoire selon la revendication 16, dans lequel chacun desdits éléments de protection (19) se présente sous la forme d'un capuchon qui peut se monter sur une pointe opérationnelle d'un élément de guidage (12, 13) respectif.

18. Dispositif médical (20, 20') pour la découpe au laser, l'ablation, la résection, la vaporisation et/ou la coagulation d'un tissu, par endoscopie, comprenant :
un accessoire (10) selon l'une quelconque des revendications 1 à 17, et
au moins un élément pour s'interfacer avec ledit accessoire (10).

19. Dispositif médical (20, 20') selon la revendication 18, comprenant en outre des seconds moyens de régulation pour réguler l'inclinaison d'au moins l'un desdits éléments de guidage.

20. Dispositif médical selon la revendication 18 ou 19, comprenant en outre un dispositif de translation.

21. Dispositif médical selon l'une quelconque des revendications 18 à 20, comprenant en outre des seconds moyens optiques pour la visualisation endoscopique.

22. Dispositif médical selon la revendication 21, dans lequel lesdits seconds moyens optiques comprennent un oculaire (33, 33') et/ou une lentille et/ou une lentille d'objectif et/ou une caméra vidéo.
